# EUROPEAN PATENT APPLICATION

(11) **EP 0 602 988 A1**
(43) Date of publication of application: **22.06.1994**
(21) Application number: 93310196.6
(22) Date of filing: 16.12.1993
(51) Int. Cl.: A61F 13/46, A41B 9/14, A41D 20/00, A61F 5/01

(54) **Multi-layer moisture management elastic fabric**

(30) Priority: 17.12.1992 US 991761; 12.01.1993 US 3263; 26.02.1993 US 23006
(71) Applicant: INTELPRO CORPORATION (a Delaware Corporation), Lincolnton North Carolina 28093 (US)
(72) Inventor: Moretz, Herbert Lynn, Davidson North Carolina 28036 (US); Brier, Daniel L., Key Largo Florida 33037 (US)
(74) Representative: Skinner, Michael Paul

(57) **Abstract**

A multi-layer moisture management elastic fabric including a moisture transport fabric layer (21,31,41,51) constructed of hydrophobic yarns and defining a first fabric face for residing in skin contact during garment wear and for wicking moisture away from the skin. A moisture dispersal fabric layer (22,32,42,52) is constructed of hydrophilic yarns and defines a second fabric face for residing in spaced-apart relation from the skin during garment wear and for receiving moisture from the hydrophobic moisture transport layer. Elastic yarns (23) are integrated with the yarns of the moisture transport fabric layer and the yarns of the moisture dispersal fabric layer to form a single, integrated fabric which is highly elastic. The fabric is useful as waistband material and as athletic headbands, wristbands and as medical bandages and braces.

## Description

This invention relates to multi-layer moisture management elastic fabrics and to garments incorporating such fabrics. As used in this application, the term "moisture management" may refer to the management of urine discharged into the garment by mild to moderately incontinent wearers, or to perspiration resulting from work, athletic activity or any other activity.

Examples of such garments are mens' boxer shorts and briefs, athletic apparel such as sweat pants, running shorts, athletic supporters, uniforms, pyjamas, bathing suits, and womens' panties, brassieres and similar undergarments, as well as medical bandages and braces, head, wrist, hat and arm bands, and the like.

The fabric from which these moisture management garments are constructed is intended to quickly move moisture away from the skin of the wearer and slow the outward movement of the moisture while at the same time enhancing the dispersion of the moisture to those fibres of the fabric which do not touch the skin. The fabric also permits gradual migration of moisture in the form of vapor to the outer surface of the fabric in a controlled manner where evaporation will occur. The result of these functions is to keep the skin as dry as possible while preventing outer clothing from becoming wet from urine or perspiration through the undergarment from inside to outside. This prevents urine or perspiration-soaked garments from residing next to the skin over a period of time. This condition can cause odour, chafing, irritation and conditions conducive to bacteria, fungus and yeast growth.

A number of problems must be solved to provide a fabric which truly manages moisture in an efficient and hygienic manner, and to incorporate that fabric into a suitable garment. Such a fabric should also take advantage of the inherent shape of the garment by moving moisture to those areas where dispersion and evaporation can most readily be accomplished, and where penetration of moisture through to the other areas of the garment and to outer clothing is minimized. In general, this involves, as disclosed herein for certain garments, moving the moisture upwardly towards the waist and away from the crotch area. The waist area has a much greater surface area than the crotch and therefore can accommodate the spreading liquid over a much large area. Of course, the problem to be solved is how to get the moisture to move upwardly against the pull of gravity. Prior applications addressed the solution to this problem. This application addresses the problem of how to prevent the moisture, once it has migrated upwardly towards the waist, from soaking into the elastic waistband of such garments and remaining in contact with the skin under the waistband, and the related problems associated with medical body wraps and athletic sweatbands.

It is an object of this invention to obviate and/or mitigate the above disadvantages.

According to one aspect of the invention, there is provided a multi-layer moisture management elastic fabric, comprising a moisture transport fabric layer constructed of hydrophobic yarns and defining a first fabric face for residing in skin contact during garment wear and for wicking moisture away from the skin, a moisture dispersal fabric layer constructed of hydrophilic yarns and defines a second fabric face for residing in spaced-apart relation from the skin during garment wear and for receiving moisture from the hydrophobic moisture transport layer, an elastic yarn integrated with the yarns of the moisture transport fabric layer and the yarns of the moisture dispersal fabric layer to form a single, integrated fabric which is highly elastic.

The hydrophobic yarns and the hydrophilic yarns of the elastic fabric may be warp knitted. Preferably, the elastic yarns are laid into the structure of the warp knitted yarns.

The hydrophobic yarn of the moisture transport layer may be chosen from the group consisting of polyester and polypropylene.

The hydrophilic yarn of the moisture dispersal fabric layer may be chosen from the group consisting of hydrophilic nylon, cotton and rayon.

The elastic yarn may be chosen from the group consisting of rubber, latex and a long chain polyurethane elastomer.

The structure of the warp knitting may be a Raschel structure.

The fabric may comprise a narrow-width elastic fabric having stretch of no less than 100 per cent in the lengthwise direction and substantially no stretch in the width direction.

The elastic fabric may comprise a waistband for a garment.

The elastic fabric may comprise a body part support wrap.

The elastic yarns may be laid into the fabric intermediate the moisture transport fabric layer and the moisture dispersal fabric layer.

According to another aspect of the invention, a warp-knitted multi-layer moisture management elastic fabric is provided, and comprises a moisture transport fabric layer constructed of hydrophobic yarns and defining a knitted first fabric face for residing in skin contact during garment wear and for wicking moisture away from the skin, a moisture dispersal fabric layer integrated by knitting with the moisture transport fabric layer, the moisture dispersal fabric layer being constructed of hydrophilic yarns and defining a knitted second fabric face obverse to the first fabric face for residing in spaced-apart relation from the skin during garment wear and for receiving moisture from the hydrophobic moisture transport layer, elastic yarns integrated by being laid in with the yarns of the moisture transport fabric layer and the yarns of the moisture dispersal fabric layer to form a single, integrated fabric which is highly elastic, the elastic fabric comprises a narrow-width elastic fabric having stretch of no less than 100 per cent in the lengthwise direction and substantially no stretch in the width direction.

The fabric is preferably no less than one inch wide and no more than two inches wide.

The hydrophobic yarns and the hydrophilic yarns of the elastic fabric may be warp knitted, and the elastic yarns are preferably laid into the structure of the warp knitted yarns.

The hydrophobic yarns of the moisture transport layer may be chosen from the group consisting of polyester and polypropylene.

The hydrophilic yarn of the moisture dispersal fabric layer may be chosen from the group consisting of hydrophilic nylon, cotton and rayon.

The elastic yarn may be chosen from the group consisting of rubber, latex and a long chain polyurethane elastomer.

The structure of the warp knitting may be a Raschel structure.

The fabric may be combined with a garment to form the waistband of the garment.

The fabric may be formed into a body wrap.

The fabric may be formed into components of a brassiere.

It is an advantage of one embodiment of the invention that it provides a moisture management garment such as an undergarment which has portions such as a waistband which are constructed from an integral multi-layer fabric which has moisture management characteristics.

It is another advantage of an embodiment of the invention that it provides a moisture management fabric which can be easily fabricated without extensive labour by producing the fabric in a single step on a textile processing machine.

It is another advantage of an embodiment of the invention that it provides a moisture management fabric which can be incorporated into the waistband, leg opening or similar area of garments, such as undergarments, pyjamas, athletic apparel and the like.

It is another advantage of an embodiment of the invention that it provides a waistband which wicks moisture away from an adjacent body surface.

It is another advantage of an embodiment of the invention that it provides a moisture management fabric which is constructed of an integral multi-layer fabric which has adjacent layers of hydrophobic and hydrophilic fabrics which exert a simultaneous push-pull effect on moisture to thereby move the moisture from one side of the adjacent layers to the other side.

It is another advantage of an embodiment of the invention that it provides a moisture-management garment constructed of an integral multi-layer fabric which incorporates one or more layers which are highly elastic.

It is another advantage of an embodiment of the invention that it provides a brassiere which includes components constructed of a moisture management fabric according to the present invention.

An embodiment of the invention will now be described by way of example only with reference to the accompanying drawings, in which:
Fig. 1 is a garment including an elastic moisture managing waistband according to an embodiment of the present invention;
Fig. 2 is a view of the back side of the garment shown in Fig. 1;
Fig. 3 is a headband made from the elastic moisture management garment according to an embodiment of the invention;
Fig. 4 is a wristband made from the elastic moisture management garment according to an embodiment of the invention;
Fig. 5 is an elbow or knee brace made from the elastic moisture management garment according to an embodiment of the invention;
Fig. 6 is a view of the inner, hydrophobic side of the elastic moisture management fabric according to a preferred embodiment of the invention;
Fig. 7 is a view of the inner, hydrophobic side of the elastic moisture management fabric in a stretched condition to show more clearly the knitted structure of the fabric;
Fig. 8 is an enlarged view of the inner, hydrophobic side of the elastic moisture management fabric in a relaxed condition;
Fig. 9 is an enlarged view of the inner, hydrophobic side of the elastic moisture management fabric in a stretched condition;
Fig. 10 is a view of the outer, hydrophilic side of the elastic moisture mangement fabric according to a preferred embodiment of the invention;
Fig. 11 is a view of the outer, hydrophilic side of the elastic moisture management fabric in a stretched condition to show more clearly the knitted structure of the fabric;
Fig. 12 is an enlarged view of the outer, hydrophilic side of the elastic moisture management fabric in a relaxed condition;
Fig. 13 is an enlarged view of the outer, hydrophilic side of the elastic moisture management fabric in a stretched condition; and
Fig. 14 is a brassiere, including elastic moisture management shoulder straps, back strap and under-cup material according to an embodiment of the present invention.

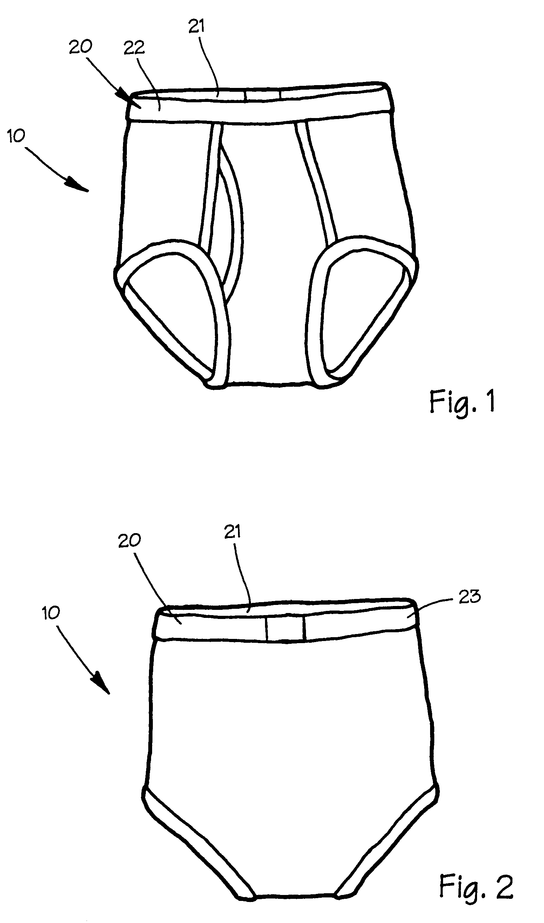

Referring now specifically to the drawings, a moisture management garment incorporating an elastic moisture management fabric according to a preferred embodiment of the invention is shown in Figs. 1 (front view) and 2 (rear view), and broadly indicated at reference numeral 10. The garment 10 is shown as a pair of men's briefs, but can be any elastic-waisted garment such as pyjamas, underwear, athletic wear, infant wear or any other apparel, such as apparel with elastic leg openings or straps, with which a moisture management elastic waistband would be useful. For purposes of example, the garment 10 may be a moisture management garment of the type disclosed in applicant's prior applications, noted above.

Whatever the garment 10, a waistband 20 according to the invention is sewn, for example, by overedge seaming stitches, to the top of the garment 10 in a conventional manner.

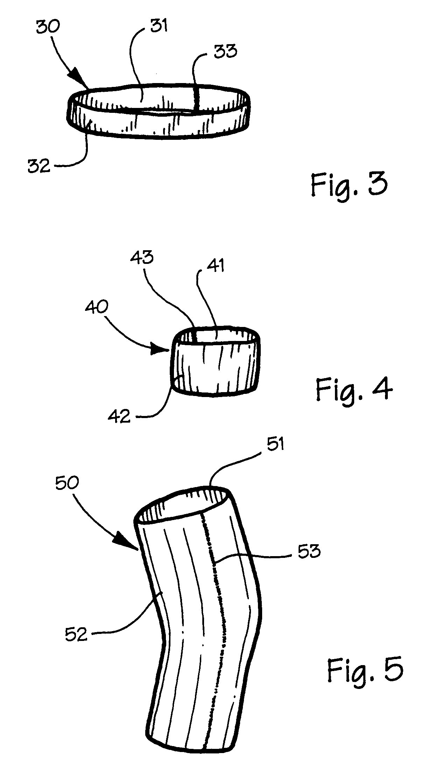

Figs. 3, 4 and 5 illustrate other embodiments of the moisture management elastic fabric claimed in this application. In accordance with the disclosure of this application, a headband 30 suitable for athletic use is shown in Fig. 3. The headband 30 is constructed of a relatively narrow moisture management elastic fabric according to the disclosure of this application. Hydrophobic inner yarns 31 and hydrophilic outer yarns 32 provide moisture management, as described below.

A wristband 40 suitable for athletic use is shown in Fig. 4. The headband 40 is constructed of a relatively wider moisture management elastic fabric according to the disclosure of this application. Hydrophobic inner yarns 41 and hydrophilic outer yarns 42 provide moisture management, as described below.

The headband 30 and wristband 40 may also include additional absorbent yarns, such as cotton terry yarns, for additional absorbency. Ordinarily these additional yarns would be placed on the outer surface of the item where the moisture thus absorbed may be more easily evaporated. The elastic yarns are selected as to number, spacing and degree of stretch to suit the needs of the particular item.

A knee or elbow brace 50 suitable for medical use is shown in Fig. 5. The knee or elbow brace 50 is constructed of a relatively wide moisture management elastic fabric according to the disclosure of this application, with relatively numerous and strong elastic yarns to provide substantial support.

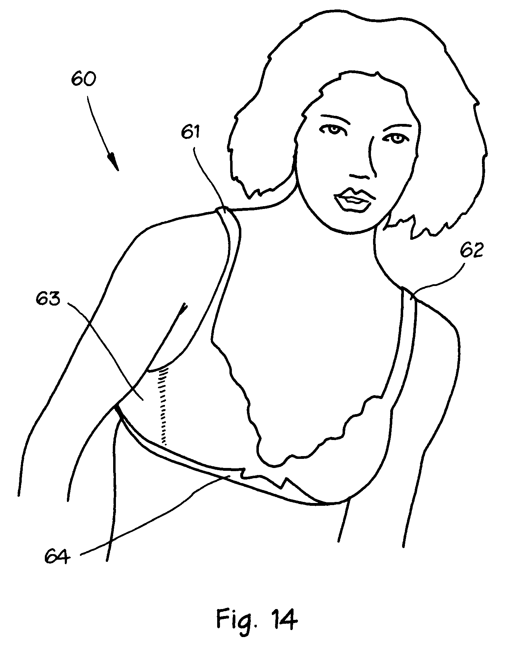

A brassiere 60 incorporating elastic moisture management fabric according to the invention is shown in Fig. 14. Brassiere 60 includes shoulder straps 61 and 62 formed of a narrow width of the fabric 20. The back strap 63 may also be formed from a wider width of moisture management fabric described in this application. In addition, under-cup elastic panel 64 may be constructed of the same fabric.

Moisture management elastic fabric 20 is described as generally representative of the fabric from which each of these structures are constructed. Moisture management fabric 20 is constructed on a warp knitting machine with weft insertion, such as the Raschelina RD3 warp knitting machine manufactured by Maschinenfabrik Jakob Muller AG, of Frick, Switzerland. Other warp knitting machines with weft insertion may also be used, as can other types of textile forming machines which can form fabrics having obverse faces of different yarns, with laid-in elastic. A desirable feature of this embodiment of the invention is the provision of hydrophobic fibres residing on the skin side of the fabric and hydrophilic fibres residing on the outer side of the fabric to provide an elastic fabric which has moisture management characteristics.

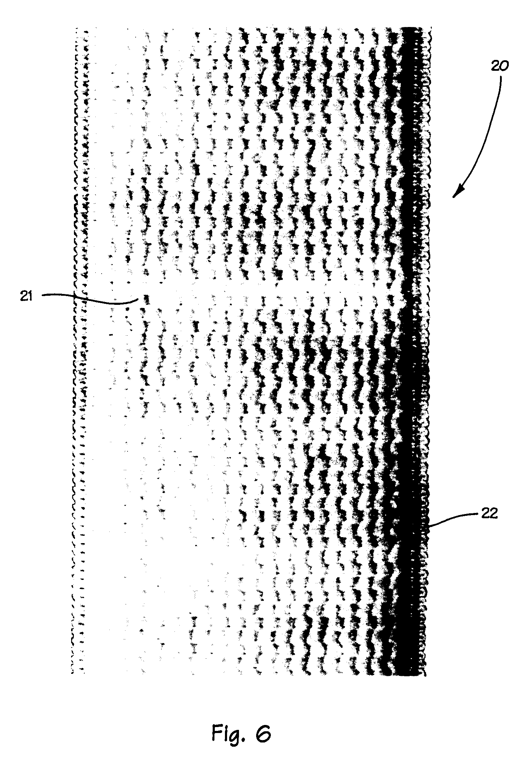

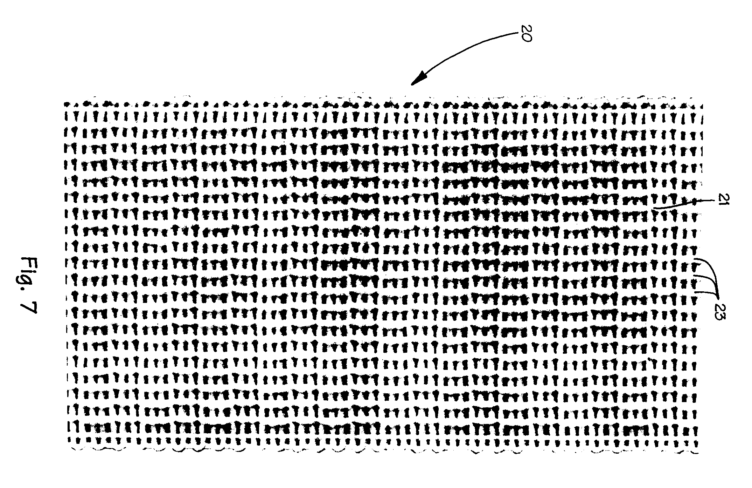

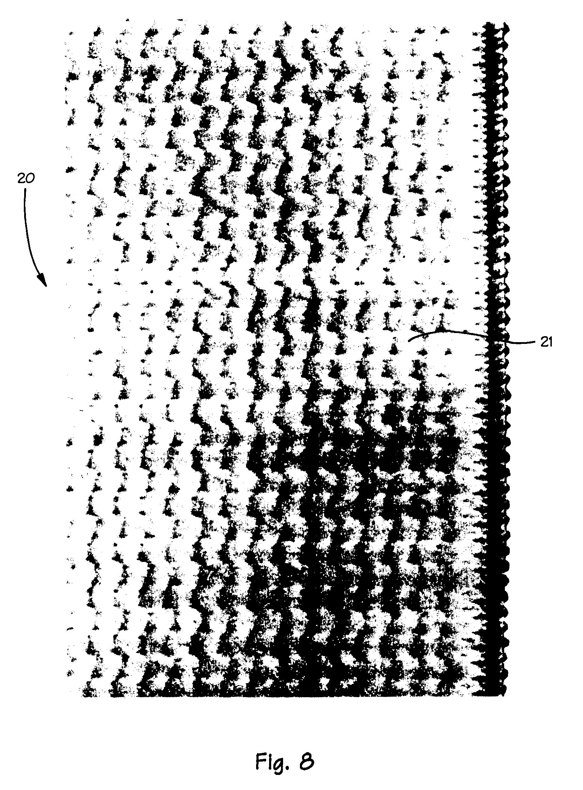

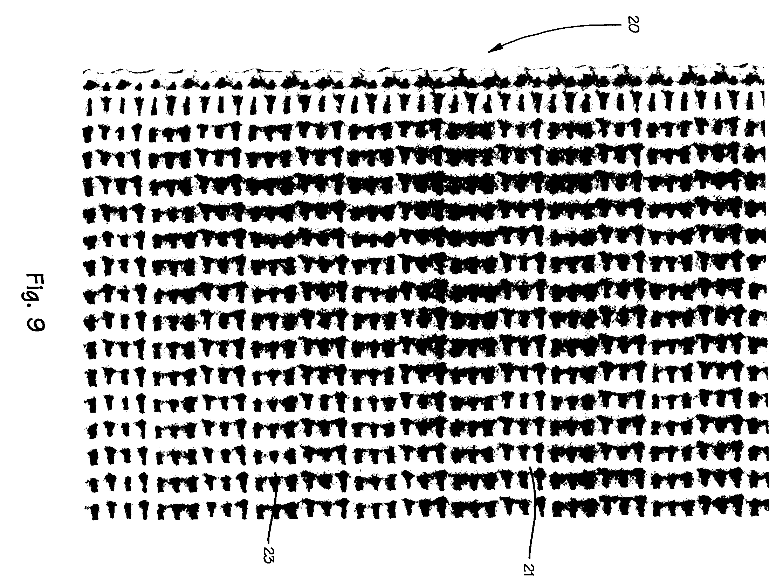

As is shown in Figs. 6-9, inner moisture transport fabric layer 21 is constructed of hydrophobic yarns formed from polyester fibre, such as fibre sold by DuPont under the trademarks Coolmax or Thermax, or from generic polyester fibres. The yarns which form the fabric layer 21 are intended to reside in skin contact during garment wear, and for this reason as many as three or four separate yarns of relatively low denier are knitted in a bulky knit to provide a soft, comfortable skin-contacting surface. These hydrophobic yarns which form the inner skin-contacting layer 21 quickly capture the moisture on the yarn surfaces and transport the moisture away from the skin through the thickness of the fabric layer 21.

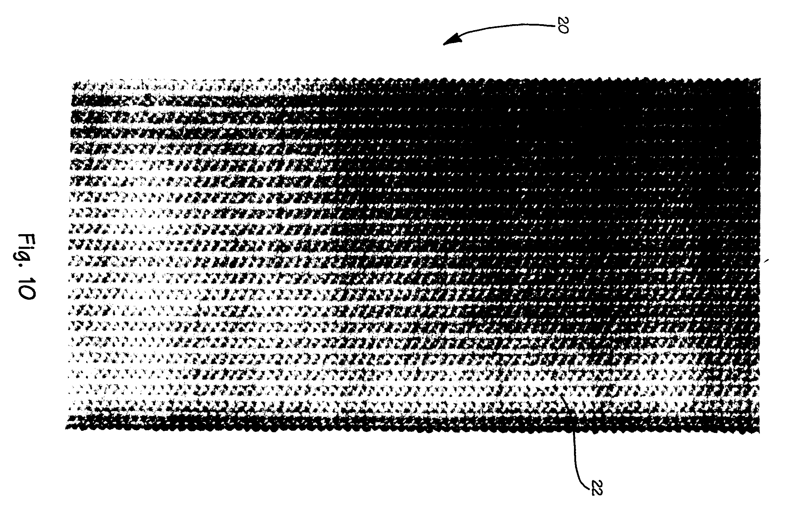

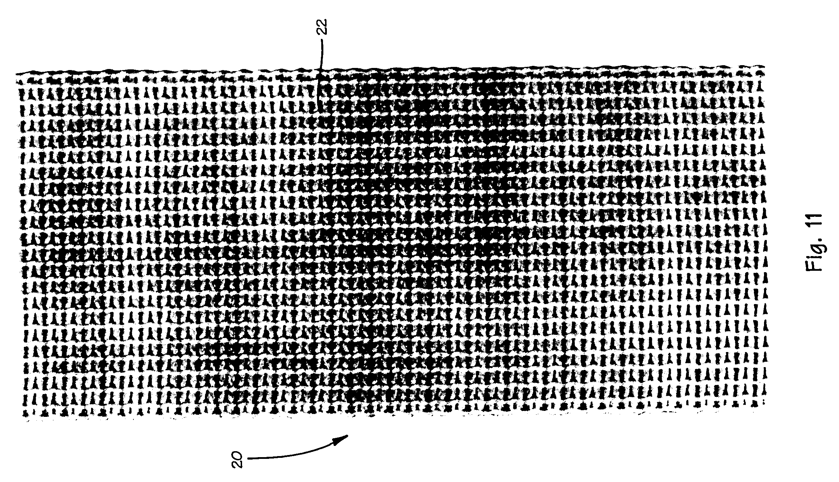

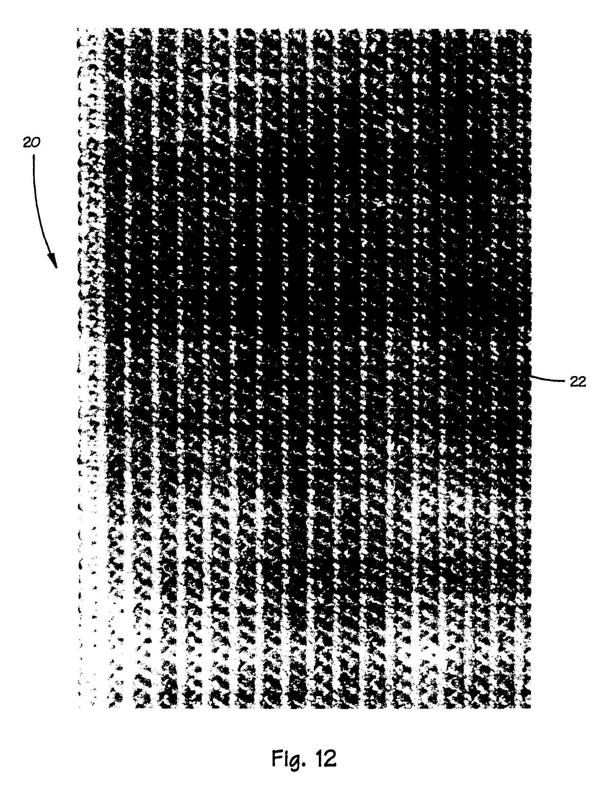

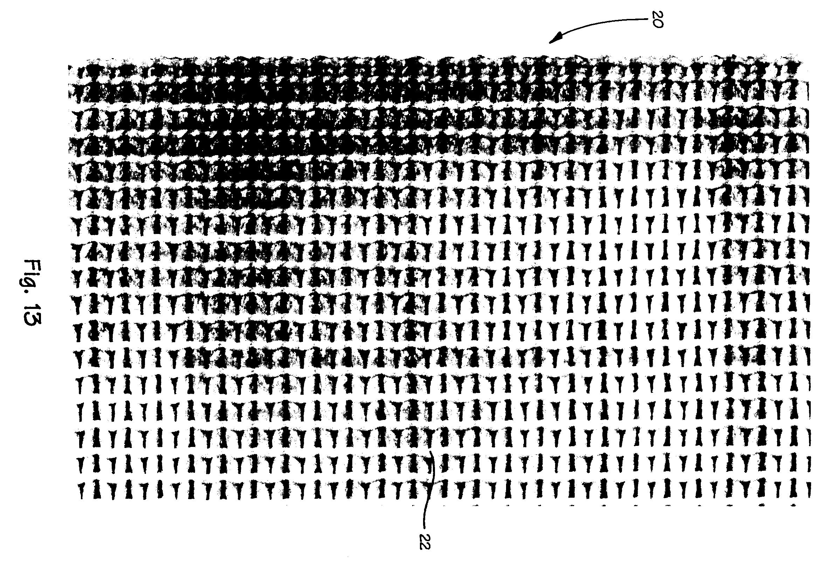

As is shown in Figs. 10-13, a moisture dispersal fabric layer 22 is constructed of hydrophilic yarns and defines a second fabric face obverse to the fabric layer 21. Fabric layer 22 is intended to reside in spaced-apart relation from the skin during garment wear. The moisture dispersal fabric layer 22 receives moisture from the hydrophobic moisture transport layer 21. The moisture dispersal fabric layer 22 receives moisture from the hydrophobic moisture transport layer 21. The moisture dispersal fabric layer 22 is constructed of hydrophilic yarns formed of fibres such as Hydrofil (R) brand fibre sold by Allied Fibres, or cotton, or blends of polyester and cotton. The fabric layer 22 is integrated with the transport layer 21 at their respective boundaries to receive and disperse moisture transported to it by the inner moisture transport layer 21. While the fabric layers 21 and 22 are distinct, they are integrated with each other and reside in contact with each other so that moisture transported from the hydrophobic transport fabric layer 21 moves into contact with the hydrophilic dispersal fabric layer 22. This results in a "push-pull" effect, whereby body heat "pushes" the moisture away from the skin along the surfaces of the hydrophobic fibres to the point where they are picked up by the fabric layer 22 and "pulled" away from the fabric layer 21 and towards the outer surface of the fabric layer 22. The "pull" results from the evaporation at the outer surface, which causes moisture in the wetter, inner areas of the fabric layer 22 to migrate towards the less wet outer areas.

The transverse diagonal loops formed by the knitting structure run in the warp direction (length) of the fabric, while the yarns running in the weft direction (width) of the fabric 20 are essentially straight. For this reason, the stretch of the fabric 20 is almost all in the warp direction of the fabric. Elastic yarns 23, such as rubber, latex or a long chain polyurethane elastomer such as Lycra brand spandex elastic, are laid-in to the structure of the fabric 20. The elastic yarns 23 run along the length of the fabric 20 and provide controlled resistance to elongating forces on the fabric 20, and quick return of the fabric 20 to its original length when the elongating forces are removed.

Moisture management fabric according to the waistband embodiment shown in Figs. 1 and 2 is approximately one to one-and-one-half inches wide (2.5 to 4 cm) and has stretch of approximately 100 per cent in the warp, or lengthwise, direction.

Moisture management fabric constructed into the headband 30 according to the embodiment shown in Fig. 3 is also approxiamtely one to one-and-one half inches wide (2.5 to 4 cm) and has stretch of approximately 50 to 100 per cent in the warp, or lengthwise, direction.

Moisture management fabric constructed into the wristband 40 according to the embodiment shown in Fig. 4 is also approximately two to three inches wide (5 to 8 cm) and has stretch of approximately 50 to 100 percent in the warp, or lengthwise, direction.

The moisture management fabric, when assembled to form a medical product such as the brace 50 according to the embodiment shown in Fig. 5, is also approximately 8 to 10 inches wide (12 to 25 cm) and has stretch of approximately 40 to 80 per cent in the warp, or lengthwise, direction.

In each case, the item 30, 40 or 50 is constructed by cutting the fabric to its suitable length and then seaming the two cut ends together with seaming stitches 33, 43, 53, respectively, to form an endless band.

In all of the embodiments disclosed above, the various fabrics can be treated with an anti-bacterial agent to retard odour and bacteria growth. Patterns and designs can also be knitted into the fabric by conventional knitting design techniques, including cross-dyeing and using different coloured and textured yarns.

A moisture management elastic fabric and several moisture management garments are described above. Various details of the invention may be changed without departing from its scope. Furthermore, the foregoing description of the preferred embodiment of the invention and the best mode for practising the invention are provided for the purpose of illustration only and not for the purpose of limitation -- the invention being defined by the claims.

Whilst endeavouring in the foregoing specification to draw attention to those features of the invention believed to be of particular importance it should be understood that the Applicant claims protection in respect of any patentable feature or combination of features hereinbefore referred to and/or shown in the drawings whether or not particular emphasis has been placed thereon.

## Claims

1. A multi-layer moisture management elastic fabric characterised by:
(a) a moisture transport fabric layer constructed of hydrophobic yarns and defining a first fabric face for residing in skin contact during garment wear and for wicking moisture away from the skin;
(b) a moisture dispersal fabric layer constructed of hydrophilic yarns and defining a second fabric face for residing in spaced-apart relation from the skin during garment wear and for receiving moisture from the hydrophobic moisture transport layer; and
(c) elastic yarns integrated with the yarns of the moisture transport fabric layer and the yarns of the moisture dispersal fabric layer to form a single, integrated fabric which is highly elastic.

2. A warp-knitted multi-layer moisture management elastic fabric, characterised by:
(a) a moisture transport fabric layer constructed of hydrophobic yarns and defining a knitted first fabric face for residing in skin contact during garment wear and for wicking moisture away from the skin;
(b) a moisture dispersal fabric layer integrated by knitting with said moisture transport fabric layer, said moisture dispersal fabric layer constructed of hydrophilic yarns and defining a knitted second fabric face obverse to the first fabric face for residing in spaced-apart relation from the skin during garment wear and for receiving moisture from the hydrophobic moisture transport layer; and
(c) elastic yarns integrated by being laid in with the yarns of the moisture transport fabric layer and the yarns of the moisture dispersal fabric layer to form a single, integrated fabric which is highly elastic;
(d) said elastic fabric comprising a narrow-width elastic fabric having stretch of no less than 100 per cent in the lengthwise direction and substantially no stretch in the width direction.

3. A moisture management elastic fabric according to Claim 11, wherein said fabric is no less than one inch wide and no more than two inches wide.

4. A moisture management elastic fabric according to Claim 1, 2 or 3, characterised in that the hydrophobic yarns and the hydrophilic yarns of said elastic fabric are warp knitted, and wherein said elastic yarns are laid into the structure of the warp knitted yarns.

5. A moisture management elastic fabric according to Claim 2, 3 or 4, characterised in that the structure of the warp knitting is a Raschel structure.

6. A moisture management elastic fabric according to Claim 2, 3, 4 or 5, characterised in that said elastic yarns are laid into the fabric intermediate the moisture transport fabric layer and the moisture dispersal fabric layer.

7. A moisture management elastic fabric according to any preceding claim, characterised in that the hydrophobic yarn of the moisture transport layer is chosen from the group consisting of polyester and polypropylene.

8. A moisture management elastic fabric according to any preceding claim, characterised in that the hydrophilic yarn of the moisture dispersal fabric layer is chosen from the group consisting of hydrophilic nylon, cotton and rayon.

9. A moisture management elastic fabric according to any preceding claim, characterised in that the elastic yarn is chosen from the group consisting of rubber, latex and a long chain polyurethane elastomer.

10. A moisture management elastic fabric according to any preceding claim, characterised in that said fabric comprises a narrow-width elastic fabric having stretch of no less than 100 per cent in the lengthwise direction and substantially no stretch in the width direction.

11. A moisture management elastic fabric according to Claim 10, characterised in that said elastic fabric comprises a waistband for a garment.

12. A moisture management elastic fabric according to Claim 10, characterised in that said elastic fabric comprises a body part support wrap.

13. A moisture management elastic fabric according to any preceding claim, in combination with a garment.

14. A moisture management elastic fabric according to any preceding claim, characterised in that the fabric is formed into a body wrap.

15. A moisture management elastic fabric according to any of Claims 1 to 15, characterised in that the fabric is formed in strap components of a brassiere.
